# EUROPEAN PATENT APPLICATION

(11) **EP 4 292 631 A1**
(43) Date of publication of application: **20.12.2023**
(21) Application number: 21925142.8
(22) Date of filing: 09.02.2021
(51) Int. Cl.: A61M 16/00, A61B 5/08, A61B 5/00

(54) **MEDICAL DEVICE SYSTEM FOR DISPLAYING MECHANICAL VENTILATION STATE**

(71) Applicant: Shenzhen Mindray Bio-Medical Electronics Co., Ltd, Shenzhen, Guangdong 518057 (CN)
(72) Inventor: ZOU, Xinru, Shenzhen, Guangdong 518057 (CN); LIU, Jinglei, Shenzhen, Guangdong 518057 (CN); WANG, Huihua, Shenzhen, Guangdong 518057 (CN); JIAO, Dongsheng, Shenzhen, Guangdong 518057 (CN); ZHOU, Xiaoyong, Shenzhen, Guangdong 518057 (CN)
(74) Representative: KIPA AB
(86) International application number: PCT/CN2021/076197
(87) International publication number: WO 2022/170461

(57) **Abstract**

A medical device system (100) for displaying a mechanical ventilation state, comprising a display (120) and a processor (110). The display (120) is used for displaying a mechanical ventilation state. The processor (110) is used for: acquiring at least one lung injury related parameter of a ventilation object; determining a lung injury risk of the ventilation object according to the at least one lung injury related parameter; and when determining that the ventilation object has a lung injury risk, controlling the display (120) to present the lung injury risk by means of preset graphics. The medical device system (100) visually presents the lung injury risk of the ventilation object by means of the preset graphics, so as to guide a user to reasonably adjust ventilation parameters, thereby maintaining ventilation while reducing lung injury.

## Description

### TECHNICAL FIELD

The disclosure relates to medical devices, and more particularly to a medical device system for displaying a state of mechanical ventilation.

### BACKGROUND

In modern clinical medicine, ventilator, as an absolutely necessary medical device for artificial replacement of spontaneous ventilation function, is widely used in medical scenarios, such as respiratory failure caused by various reasons, anesthesia and respiratory management during major surgery, respiratory support treatment and emergency resuscitation, and plays a very important role in the field of modern medicine.

Mechanical ventilation is a ventilation method that uses a mechanical device to replace, control, or change a spontaneous respiratory movement with the help of a ventilator. Mechanical ventilation can maintain airway patency, improve ventilation and oxygenation, prevent hypoxia and carbon dioxide accumulation in a body, make it possible for the body to experience respiratory failure caused by underlying diseases, and create conditions for the treatment of underlying diseases.

However, during the mechanical ventilation, improper setting of the ventilator causes lung injury to the ventilated object, and medical personnel usually need to adjust a ventilator parameter based on a monitored parameter during the mechanical ventilation. However, considering the complexity of the system, a single monitored parameter is not comprehensive enough, making it difficult to accurately and sensitively predict the safety of mechanical ventilation. Moreover, excessive and complex monitoring parameters make it difficult for user to screen useful information and make a reasonable determination.

### SUMMARY

A series of simplified concepts are introduced into summary of this disclosure, and are further elaborated in specific embodiments of this disclosure. The summary of this disclosure does not attempt to define key and necessary technical features of the claimed technical solution, nor attempt to determine a protection scope of the claimed technical solution.

A first aspect according to an embodiment of this disclosure provides a medical device system for displaying a state of mechanical ventilation, including a display and a processor; wherein the display is configured for displaying the state of mechanical ventilation;
the processor is configured for:
acquiring at least one parameter of a ventilated object, which parameter is related to lung injury of the ventilated object;
determining a risk of lung injury for the ventilated object according to the at least one parameter related to lung injury; and
controlling the display to present, through a preset graphic, the risk of lung injury, when the ventilated object is determined to be at the risk of lung injury.

A second aspect according to an embodiment of this disclosure provides a medical device system for displaying a state of mechanical ventilation, including a display and a processor; wherein the display is configured for displaying the state of mechanical ventilation;
the processor is configured for:
controlling the display to display, on a display interface, a dynamic pulmonary graphic, wherein the dynamic pulmonary graphic is configured for characterizing a state of mechanical ventilation of a ventilated object during the mechanical ventilation;
acquiring a state of diaphragm injury of the ventilated object; and
controlling the display to display, below the dynamic pulmonary graphic, a diaphragmatic graphic, wherein the diaphragmatic graphic is configured for characterizing the state of diaphragm injury.

A third aspect according to an embodiment of this disclosure provides a medical device system for displaying a state of mechanical ventilation, including a display and a processor; wherein the display is configured for displaying the state of mechanical ventilation;
the processor is configured for:
controlling the display to display, on a display interface, a dynamic pulmonary graphic, wherein the dynamic pulmonary graphic is configured for characterizing a state of mechanical ventilation of a ventilated object during the mechanical ventilation; wherein the state of mechanical ventilation of the ventilated object at least includes an abnormality of airway and/or a treatment suggestion for the abnormality of airway.

The medical device system for displaying a state of mechanical ventilation, according to embodiments of this disclosure, visualizes the risk of lung injury for the ventilated object through a preset graphic, thereby guiding the user to reasonably adjust ventilation parameter and maintain ventilation while reducing the lung injury.

### BRIEF DESCRIPTION OF THE DRAWINGS

In order to provide a clearer explanation of technical solutions in embodiments of this disclosure, a brief introduction is given to the accompanying drawings required in the description of the embodiments. It is evident that the accompanying drawings in the following description are only some embodiments of this disclosure. For ordinary technical personnel in the art, other accompanying drawings can be obtained based on these drawings without any creative effort.
FIG. 1 is a schematic block diagram of a medical device system according to an embodiment of this disclosure.
FIG. 2 is a schematic diagram of a display interface of a medical device system according to an embodiment of this disclosure.
Fig. 3 is a schematic diagram of a dynamic pulmonary graphic according to an embodiment of this disclosure.
FIG. 4 is a schematic diagram of a dynamic pulmonary graphic including a diaphragmic graphic according to an embodiment of this disclosure.
Fig. 5 is a schematic diagram of a dynamic pulmonary graphic including a dynamic thoracic graphic according to an embodiment of this disclosure.

### DESCRIPTION OF THE PREFERRED EMBODIMENTS

In order to make the purpose, technical solution, and advantages of this disclosure more obvious, the following refers to the attached drawings to describe in detail the exemplary embodiments according to this disclosure. Obviously, the described embodiments are only some of the embodiments of this disclosure, not all of them. It should be understood that this disclosure is not limited by the embodiments described here. Based on the embodiments described in this disclosure, all other embodiments obtained by those skilled in the art without creative work, fall within the protection scope of this disclosure.

In the following description, a large number of specific details are provided to provide a more thorough understanding of this disclosure. However, it is evident to those skilled in the art that this disclosure can be implemented without the need for one or more of these details. In other examples, in order to avoid confusion with this disclosure, some well-known technical features in this field are not described.

It should be understood that this disclosure can be implemented in different forms and should not be limited to the embodiments proposed here. On the contrary, providing these embodiments make the disclosure thorough and complete, and fully convey the scope of this disclosure to those skilled in the art.

The purpose of the terminology used here is only to describe specific embodiments and is not a limitation of this disclosure. When used here, the singular forms of "an", "a", and "the/said" are also intended to include the plural form, unless the context clearly indicates another way. It should also be understood that the terms, such as "comprising", "and/or", "including", when used in this disclosure, determine the presence of the features, integers, steps, operations, components, and/or elements, but do not exclude the presence or addition of one or more other features, integers, steps, operations, components, and/or elements. When used here, the term "and/or" includes any and all combinations of related listed items.

In order to fully understand this disclosure, a detailed structure is provided in the following description to explain the technical solution proposed in this disclosure. The optional embodiments of this disclosure are described in detail below, however, in addition to these detailed description, this disclosure may also have other embodiments.

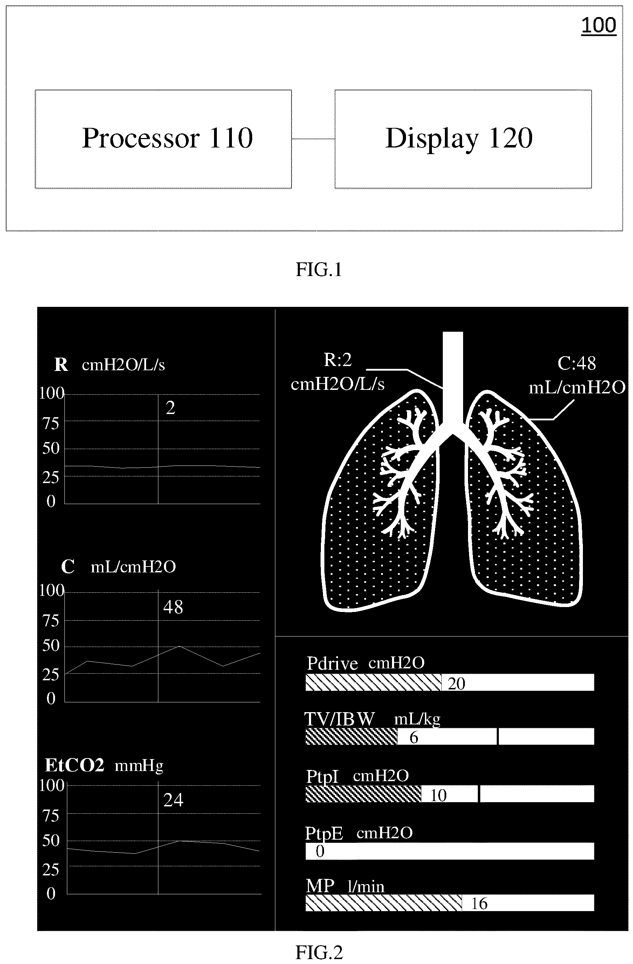

Below, a medical device system for displaying a state of mechanical ventilation according to an embodiment of this disclosure is described with reference to FIG. 1. FIG. 1 is a schematic block diagram of a medical device system 100 according to an embodiment of this disclosure.

As shown in FIG. 1, the medical device system 100 for displaying a state of mechanical ventilation includes a processor 110 and a display 120. The display 120 is configured for displaying the state of mechanical ventilation; and the processor is configured for: acquiring at least one parameter of a ventilated object, which parameter is related to lung injury of the ventilated object; determine a risk of lung injury for the ventilated object according to the at least one parameter related to lung injury; and controlling the display to present, through a preset graphic, the risk of lung injury, when the ventilated object is determined to be at the risk of lung injury. According to the embodiment of this disclosure, the medical device system 100 evaluates the risk of lung injury for the ventilated object through single or multiple parameters related to lung injury, and presents an overall risk of lung injury with a preset graphic, thus eliminating the requirement for the user to screen and analyze each parameter separately. By referring to the related information on the risk of lung injury presented by the preset graphic, the user can reasonably configure parameters, such as pressure, volume, and frequency, to provide the mechanical ventilation for the ventilated object while avoiding the risk of lung injury.

Wherein, the ventilated object can specifically refer to a patient who needs to use a mechanical ventilation device (such as a ventilator) for respiration, due to respiratory failure or difficulty in spontaneous respiration. User of the medical device system 100 can be medical staff who views the ventilation state of the ventilated object. The medical device system 100 is implemented as a ventilation device, which provides mechanical ventilation to the ventilated object, such as a ventilator and other ventilation devices. When a respiratory system of the ventilated object is unable to complete normal spontaneous respiration, mechanical ventilation is provided through the ventilator to provide respiratory support. During mechanical ventilation, monitoring parameters for ventilation can be acquired through a corresponding monitoring device. The medical device system 100 is also implemented as an electronic device other than ventilator, such as a central station for ventilator. The processor 110 of the central station acquires real-time parameters related to lung injury through a communication interface, determines the risk of lung injury for the ventilated object according to the parameters related to lung injury, and controls the display 120 to display the risk of lung injury through the preset graphic.

The processor of the medical device system 100 can obtain at least one parameter related to lung injury in real-time during the mechanical ventilation. The parameter related to lung injury in the embodiment of this disclosure can be various parameters directly or indirectly related to lung injury, and can include at least one of: a control parameter for ventilation and a monitoring parameter for ventilation. The monitoring parameter for ventilation can reflect a state of the ventilated object, while the control parameter for ventilation can reflect a state of the ventilation device. Both the monitoring parameter and the control parameter can reflect and affect the risk of lung injury.

Wherein, the monitoring parameter for ventilation includes at least one of: an airway plateau pressure, a driving pressure, a monitoring value for positive end expiratory pressure (PEEP), a monitoring value for tidal volume (TV), a functional residual volume, a transpulmonary pressure, mechanical power, respiratory power and an esophageal pressure.

Specifically, the airway plateau pressure refers to an end-inspiratory airway pressure when an air flow pauses, representing an average level of alveolar pressure at an inspiratory end. The main factor affecting the airway plateau pressure includes tidal volume, compliance of lung(s) and PEEP. During the mechanical ventilation, if the airway plateau pressure is too high, the probability of barotrauma significantly increases. Compared to a peak airway pressure, the airway plateau pressure can better reflect the risk of barotrauma, because the airway peak pressure mainly acts on the airway, while the airway plateau pressure truly reflects the maximum pressure inside the alveoli. The airway plateau pressure can effectively assess the risk of excessive lung dilation.

The driving pressure is a difference between the plateau pressure and PEEP, which represents the driving pressure for expansion and dilation of the respiratory system and is closely related to lung dilation at an inspiratory phase. Driving pressure can be expressed as a ratio of VT to compliance of respiratory system. Due to the corresponding relationship between the compliance of respiratory system and inflation capacity of lungs, the driving pressure can be understood as a ratio of VT to total lung capacity, which is related to strain of lungs. Driving pressure includes a driving pressure for the respiratory system, a transpulmonary driving pressure, etc. Transpulmonary driving pressure is a difference value between a value, which is obtained by the airway plateau pressure minus PEEP, and a value, which is obtained by the esophageal plateau pressure minus end-expiratory esophageal pressure. When considering the compliance of chest wall, the transpulmonary driving pressure can better reflect the pulmonary pressure. The airway driving pressure is a difference between the plateau pressure and PEEP, and represents an impact of cyclic strain on the lung parenchyma during each ventilation cycle. The difference between the transpulmonary driving pressure and the airway driving pressure is mainly due to an increase in the compliance of chest wall. The difference the between the airway driving pressure and the transpulmonary driving pressure is mainly related to an individual situation of the ventilated object. For the ventilated object without spontaneous respiration, the transpulmonary driving pressure is usually lower than the airway driving pressure.

The monitoring value for positive end expiratory pressure (PEEP) is a positive pressure retained in a respiratory tract at the end of the respiratory cycle (expiratory end), and is greater than an atmospheric pressure of the ventilated object. A certain positive end expiratory pressure can help lung recruitment and dilate collapsed alveoli, while a lower PEEP may cause airway collapse. Therefore, the monitoring value for positive end expiratory pressure can be configured to evaluate a lung collapse injury.

The monitoring value for tidal volume (TV) refers to a volume of air inhaled or exhaled each time when breathing calmly. Whether the tidal volume is appropriate is related to the individual situation of the ventilated object. For example, for a patient with pulmonary disease, his/her normal pulmonary tissue decreases, and the corresponding ventilation capacity of such decreased pulmonary tissue is far less than that of normal pulmonary tissue. Therefore, even if a small tidal volume is given for mechanical ventilation, the pulmonary area of the patient with pulmonary disease, which pulmonary area contains air, can be excessively dilated, resulting in atelectasis, mediastinal emphysema, pulmonary edema and other injuries. After the processor 110 acquires the monitoring value for tidal volume, it can specifically calculate the ratio of the monitoring value for tidal volume to ideal body weight (TV/IBW), or determine the risk of lung injury by monitoring the tidal volume/functional residual volume.

Functional residual volume refers to an amount of gas remaining in the lungs after a calm exhalation, which is equal to the sum of an expiratory reserve volume and a residual volume. An increase in the functional residual volume indicates alveolar dilation, while a decrease in functional residual volume indicates alveolar contraction and alveolar closing. The functional residual volume plays a buffering role in physiologically stabilizing the alveolar partial pressure, and reduces the impact of gas exchange in the alveoli during a ventilation interval. If there is no functional residual volume, the alveoli is completely closed at the expiratory end.

Transpulmonary pressure refers to a difference between the alveolar pressure and intrathoracic pressure, which is the force that causes the alveolar dilation and contraction. Transpulmonary pressure can quantitatively represent the mechanical pressure of the lungs during ventilation. In clinical practice, the airway pressure can be measured instead of alveolar pressure, and the esophageal pressure can be measured instead of intrathoracic pressure. The measurement position of esophageal pressure is in a middle section of the esophagus, where the esophageal pressure can accurately reflect the intrathoracic pressure. Usually, it is necessary to measure at the two important time points of the respiratory process: inspiratory end (to avoid excessive lung dilation) and expiratory end (to avoid excessive lung collapse). Therefore, the monitoring value for the transpulmonary pressure mainly includes end-inspiratory transpulmonary pressure and end-expiratory transpulmonary pressure. Excessive dilation of pulmonary tissue is a key factor causing lung injury, and the transpulmonary pressure can be configured to monitor the local excessive dilation of pulmonary tissue. Inappropriate application of high inspiratory pressure and large tidal volume may increase transpulmonary pressure, increase alveolar hyperinflation, and aggravate lung injury.

The essence of mechanical power is similar to that of respiratory power. The respiratory power is power transmitted by the ventilator to the respiratory system to complete the pulmonary ventilation. It is an integral of a changed part of inspiratory pressure and tidal volume, and presents the kinetic energy part, excluding the potential energy part generated by the unchanged positive end expiratory pressure and tidal volume during the ventilation. Respiratory power can be obtained by accumulation of unit time or unit volume conversion of units. Respiratory power is the kinetic energy part of the energy transmitted by the ventilator to the respiratory system. On this basis, the mechanical power further includes the potential energy part of the integral of positive end expiratory pressure and tidal volume that does not change during ventilation. Similarly, the mechanical power can also be calculated from tidal volume, airway pressure and respiratory rate. The state of the lungs themselves (end-expiratory pulmonary volume, compliance, airway resistance) is also a factor affecting the mechanical power. When the same tidal volume is applied to the lungs in different states, different power is required. The mechanical power generated by single ventilation is equal to a product of inspiratory pressure and tidal volume, which is actually the integral of the inspiratory pressure and tidal volume, namely an area under a pressure-volume curve. When the airway pressure is used as the inspiratory pressure, the mechanical power obtained is the mechanical power of the respiratory system (MPrs). When the transpulmonary pressure is used as the inspiratory pressure, the mechanical power obtained is a transpulmonary mechanical power (MPlung). The mechanical power generated per unit time is the product of the mechanical power of single ventilation and the respiratory rate, or a sum of all ventilation mechanical power per unit time.

Esophageal pressure can be configured to assess a pulmonary tension and thus assessing the risk of lung injury. Due to that the thoracic cavity is closed, the esophageal pressure can be configured to estimate the intrathoracic pressure and further calculate the monitoring parameter for ventilation, such as transpulmonary pressure. Processor 110 can also use floated values of the esophageal pressure to assess the risk of lung injury.

In addition to the above monitoring parameter for ventilation, the control parameter for ventilation can also be configured to assess the risk of lung injury. Inappropriate setting of the control parameter for ventilation increases the possibility of risk of lung injury, as the control parameter for ventilation can also reflect the risk of lung injury. Control parameter for ventilation includes at least one of: a setting value for tidal volume (TV); an ideal weight and a height; a setting value for inspiratory pressure, and a setting value for positive end expiratory pressure (PEEP).

When determining the risk of lung injury for a ventilated object according to at least one parameter related to lung injury, one implementation method is that the ventilated object is determined to be at the risk of lung injury when at least one parameter related to lung injury satisfies a first preset rule. For example, as long as one parameter related to lung injury satisfies the first preset rule, it represents there is a possibility of lung injury. Therefore, it is determined that the ventilated object is at the risk of lung injury, and the user is presented, through a preset graphic, with information about the risk of lung injury for the ventilated object, thereby reducing the possibility of report missing. Alternatively, in order to reduce the possibility of false reporting, the processor 110 can determine the risk of lung injury for the ventilated object when two or more parameters related to lung injury satisfy the first preset rule.

Wherein, at least one parameter related to lung injury satisfies the first preset rule, includes: at least one parameter related to lung injury exceeds a corresponding preset threshold. The preset threshold corresponding to the parameter related to lung injury can be a numerical point or a certain numerical range. The preset threshold can be determined by the processor 110, and can be a fixed value or floated value. The processor 110 can adopt a fixed preset threshold or adjust the preset threshold in real-time based on individual situation and ventilation state of the ventilated object. The preset threshold can also be set by the user, for example, the user can set the threshold corresponding to each parameter related to lung injury through a setting option displayed on the display interface. In addition, the first preset rule can also be implemented as other rules, for example, the processor 110 can determine whether the parameter related to lung injury satisfies the first preset rule based on a trend of the parameter related to lung injury over a period of time.

Optionally, the processor 110 can also combine at least two parameters related to lung injury to determine whether the ventilated object is at the risk of lung injury. For example, the processor 110 can perform a weighted summation or other calculation on at least two parameters related to lung injury, and determine whether the ventilated object is at the risk of lung injury according to the calculation result.

According to the different mechanisms of lung injury, the types of lung injury mainly include alveolar collapse (lung collapse injury) and excessive dilation (pressure injury, volume injury). The pressure injury mainly includes tension pneumothorax, mediastinal emphysema, etc., which are caused by excessive airway pressure. Ventilation at increasing pulmonary volume can cause excessive dilation of local pulmonary tissue, leading to the pressure injury. The formation of volume injury is mainly related to excessive traction of alveolar epithelium and vascular endothelium by excessive end-inspiratory pulmonary volume. Mechanical ventilation at low pulmonary volume can also cause lung injury. The mechanism of this lung injury includes repeated opening and closing of airway and lung unit, change of surfactant function and local hypoxia. This kind of lung injury is called as lung collapse injury.

Different monitoring parameters for ventilation or different control parameters for ventilation can reflect different types of lung injury. For example, the volume injury is related to tidal volume/ideal body weight (TV/IBW), the pressure injury is related to the driving pressure, and the lung collapse injury is related to positive end expiratory pressure (PEEP). Therefore, different monitoring parameters for ventilation or different control parameters for ventilation can be used as indicators to evaluate degrees of different types of lung injury. Comprehensive assessment of lung injury with multiple parameters can comprehensively and accurately determine the risk of lung injury for the ventilated object. At the same time, specific types of the risk of lung injury can also be evaluated.

After obtaining information about the risk of lung injury, the processor 110 is further configured to control the display 120 to display the preset graphic on a display interface to present the risk of lung injury. Based on the preset graphic, the user can intuitively understand the risk of lung injury for the ventilated object under the current setting condition of the ventilator, and then decide whether to adjust the ventilator parameter, and with the assistance of the preset graphic, make reasonable parameter setting. The preset graphic can present information, such as whether the ventilated object is at the risk of lung injury, the type of the risk of lung injury, and the degree of the risk of lung injury, as detailed in the followings. The specific shape of the preset graphic can be selected according to actual requirement, as long as it can serve as a carrier for dynamically presenting the risk of lung injury. By way of example, presenting the risk of lung injury through the preset graphic includes presenting the risk of lung injury through at least one graphic feature of color, brightness, and transparency in the preset graphic. In some embodiments, the risk of lung injury can also be presented by preset graphic features, such as shape and size.

The processor 110 can control the display 120 to display the preset graphic in a preset display area of the display 120 in real time, that is, the display 120 can display the preset graphic in a fixed area of a home interface as part of the home interface. Alternatively, the processor 110 can also control the display 120 to display the preset graphic on a pop-up window of the display 120 when an instruction for displaying the preset graphic is received. That is, the preset graphic is displayed as a tool. Processor 110 can also automatically control the display 120 to display the preset graphic when determining that the ventilated object is at the risk of lung injury. The embodiments of this disclosure do not limit this.

Preferably, the preset graphic can be a preset dynamic graphic, which changes in real time according to a state of the parameter related to lung injury, thus presenting the risk of lung injury in real time, so that the user can learn about the risk of lung injury by referring to the preset dynamic graphic, and adjust the ventilator parameter in real time.

The preset dynamic graphic can be realized as a dynamic pulmonary graphic, and the display 120 can present the risk of lung injury through the dynamic pulmonary graphic. Referring to FIG. 2, on the display interface shown in FIG. 2, the display 120 displays the dynamic pulmonary graphic at top right of the display interface. Dynamic pulmonary graphic are intuitive and visual, which can help the user to understand the risk of lung injury. Specifically, the dynamic pulmonary graphic can be drawn according to a shape of lungs of a human body, for example, it can vividly include left and right lung lobes, trachea, and left and right bronchi located in the left and right lung lobes. Dynamic pulmonary graphic are not limited to a two-dimensional plane graphic, but also a three-dimensional graphic.

Presenting the risk of lung injury through the dynamic pulmonary graphic can include any of the following two forms: presenting the risk of lung injury through a graphic feature inside the dynamic pulmonary graphic, and presenting the risk of lung injury through a graphic feature inside a background area of the dynamic pulmonary graphic. Taking the graphic feature as a color feature for example, the display 120 can present the risk of lung injury not only through the color inside the dynamic pulmonary graphic, but also through the color inside the background area of the dynamic pulmonary graphic. The color inside the dynamic pulmonary graphic can be the color of a contour of the dynamic pulmonary graphic, or the color of an area inside the contour of the dynamic pulmonary graphic.

When presenting whether the ventilated object is at the risk of lung injury through the preset graphic, the processor 110 can control the display 120 to present a first graphic feature of the preset graphic, if it is determined that the ventilated object is at the risk of lung injury, and control the display 120 to present second graphic feature of the preset graphic, if it is determined that the ventilated object is free of the risk of lung injury. The user can intuitively determine that the ventilated object is at the risk of lung injury by determining whether the graphics have the first graphic feature.

Wherein, the first and second graphic features can include different color features, brightness features, grayscale features, or specific graphic or textual marks. For example, when the first graphic feature and the second graphic feature are color features, red can be configured to indicate the risk of lung injury. When the processor 110 determines that the ventilated object is at the risk of lung injury, it controls the display 120 to display the preset graphic in red. When the processor 110 determines that the ventilated object is free of the risk of lung injury, it controls the display 120 to display the preset graphic in other colors. In practical applications, the type or types of graphic features configured to present the risk of lung injury can be designed as needed, without specific limitations here.

In one embodiment, if the processor 110 determines that the ventilated object is free of the risk of lung injury, it can also control the preset graphic to change with respiratory state. The preset graphic is displayed in white during normal inhalation and in blue during normal exhalation.

In one embodiment, when it is determined that the ventilated object is at the risk of lung injury, the display 120 can also reflect a corresponding relationship between the risk of lung injury and the parameter related to lung injury, through the preset graphic, enabling the user to understand which parameter related to lung injury causes the risk of lung injury. Specifically, the processor 110 can control the display 120 to display the graphic feature of the preset graphic, which feature corresponds to the parameter related to lung injury, according to the corresponding relationship between the parameter related to lung injury and the graphic feature of the preset graphic, thereby guiding the user to adjust the parameter related to lung injury. For example, if the driving pressure exceeds the preset threshold, and causes the processor 110 to determine that the ventilated object is at the risk of lung injury, the preset graphic is presented in one color. If the mechanical power exceeds the preset threshold and causes the processor 110 to determine that the ventilated object is at the risk of lung injury, the preset graphic is presented in another color. The user can distinguish which parameter related to lung injury exceeds the preset threshold according to the color of the preset graphic, enable the processor 110 to determine that the ventilated object is at the risk of lung injury, and then eliminate the risk according to this parameter.

In the corresponding relationship between the aforementioned parameter related to lung injury and the graphic feature of the preset graphic, each parameter related to lung injury can correspond to one graphic feature, or each type of parameter(s) related to lung injury can correspond to one graphic feature. The parameters related to the same type of lung injury can be parameters related to the same type of lung injury. Alternatively, the same type of parameter related to lung injury can also be different parameters related to lung injury with the same properties, for example, end-inspiratory transpulmonary pressure and end-expiratory transpulmonary pressure that require the esophageal pressure to be monitored, can be classified as the same type of parameters related to lung injury.

The corresponding relationship between the parameter related to lung injury and the graphic feature of the preset graphic can include at least one of: a corresponding relationship between the parameter related to lung injury and a graphic feature of a whole area of the preset graphic, as well as a corresponding relationship between the parameter related to lung injury and a graphic feature of a partial area of the preset graphic. If the ventilated object is determined to be at the risk of lung injury according to at least one parameter related to lung injury, the preset graphic can be presented as a whole according to the corresponding relationship between the parameter related to lung injury and the graphic feature of the whole area of the preset graphic, so as to present information, such as whether the ventilated object is at the risk of lung injury, a degree of the risk of lung injury, and a type of the risk of lung injury. Alternatively, it is possible to determine a specific area where the lung injury may occur according to a certain parameter related to lung injury, and present, through a graphic feature of a partial area, information such as whether the ventilated object is at the risk of lung injury in the partial area, the degree of the risk of lung injury, and the type of the risk of lung injury, according to the corresponding relationship between the parameter related to lung injury and the graphic feature of the partial area.

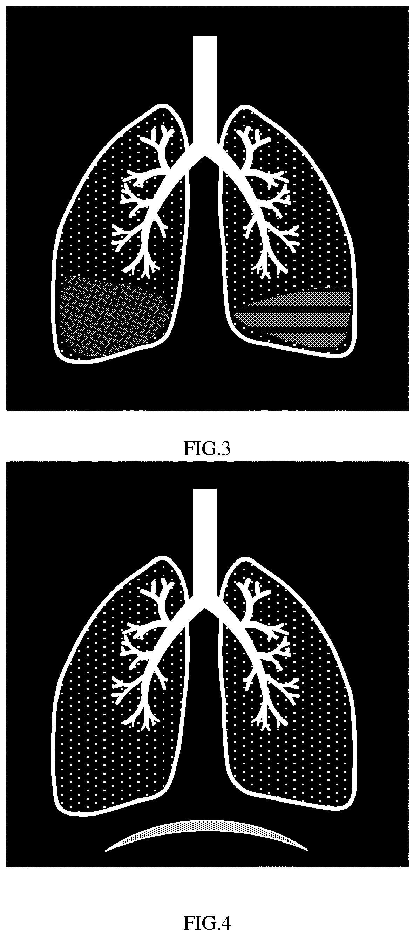

For example, the end-expiratory transpulmonary pressure can be configured to determine whether the patient is at the risk of lung injury or the risk of alveolar collapse during the exhalation phase of the ventilation state. For example, as shown in FIG. 3, a monitoring value for end-expiratory transpulmonary pressure (PtpE) is -8 cmH2O, which is less than the corresponding preset threshold range (-5~5cmH2O), indicating that the patient is at the risk of end-expiratory alveolar collapse. Therefore, the processor 110 can control the display 120 to display an area with the risk of alveolar collapse at a lower part of the dynamic pulmonary graphic, and the degree of risk for alveolar collapse can be reflected through a graphic feature, such as a size or shade of color of the area with the risk of alveolar collapse.

In one embodiment, the processor 110 can also control the display 120 to display proportions of various types of lung injury in the risk of lung injury, such as pressure injury, volume injury, collapse injury, energy injury, etc. The specific display method includes numerical value, text, graphic, etc. Display 120 can display the proportions of various types of lung injury in the risk of lung injury through the preset graphic, or display proportions of various types of lung injury in the risk of lung injury through other graphic, text, or numerical value besides the preset graphic.

In one embodiment, the processor 110 is further configured to determine the degree of the risk of lung injury according to the parameter related to lung injury and present the degree of the risk of lung injury through the preset graphic. For example, the severer the parameter related to lung injury exceeds the preset threshold, the higher the degree of risk of lung injury, or the more the parameters related to lung injury exceed the preset threshold, the higher the degree of risk of lung injury. The processor 110 can also assess the degree of the risk of lung injury according to other criteria.

For example, presenting the degree of the risk of lung injury through the preset graphic includes displaying the preset graphic corresponding to different degrees of the risk of lung injury through at least one of: different colors, different shades of color, and different transparencies. For example, when the degree of the risk of lung injury is represented by different shades of color, the higher the degree of the risk of lung injury, and the darker the color of the preset graphic. When the degree of the risk of lung injury is represented by different colors, as the degree of the risk of lung injury increases from low to high, the color of the preset graphic gradually changes from one color to another, for example, from blue to red. In other embodiments, the degree of the risk of lung injury can also be reflected in text or symbol in the preset graphic.

In one embodiment, the processor 110 is further configured to control the display 120 to display at least one parameter related to lung injury in an adjacent area of a preset graphic, through at least one form of numerical value or chart. Adjacently displaying the preset graphic that presents the risk of lung injury and the parameter related to lung injury, is beneficial for the user to promptly view the parameter related to lung injury when knowing that the ventilated object is at the risk of lung injury.

Wherein, the display 120 can always display the parameter related to lung injury adjacent to the preset graphic on the same screen. Alternatively, the processor 110 can control the display 120 to display the parameter related to lung injury in an adjacent area of the preset graphic upon receiving an instruction for displaying the parameter related to lung injury. For example, the user can click on the preset graphic or other options to invoke the parameter related to lung injury for viewing when viewing the preset graphic which indicates the ventilated object is at the risk of lung injury.

The parameter related to lung injury displayed on display 120 can be all or some of parameters related to lung injury. When displaying some parameters related to lung injury, the parameters related to lung injury displayed on the display 120 can be the parameters related to lung injury that enable the processor 110 to determine that the ventilated object is at the risk of lung injury. Alternatively, the parameter related to lung injury displayed on the display 120 can be the parameter that user is more concerned about. User can set an option to preset which parameter related to lung injury is displayed on the display 120.

As shown in FIG. 2, the display 120 can display the parameter related to lung injury below the dynamic pulmonary graphic. Of course, the relative position relationship between the two is not limited to this, as long as the display 120 displays the dynamic pulmonary graphic and parameter related to lung injury on the same screen. As an example, FIG. 2 shows the value of each parameter related to lung injury in a progress bar combined with numerical values, but it can be understood that the presentation form of parameter related to lung injury is not limited to this, for example, the parameter related to lung injury can also be shown in the form of bar graphic, mesh graphic, broken line graphic, point graphic, etc. In practical applications, the form of presenting parameter related to lung injury can be designed according to requirements, without specific limitations here.

The processor 110 is further configured to control the display 120 to display the state of parameter related to lung injury, and the presentation method can include at least one form of numerical value and chart. Wherein, the state of parameter related to lung injury includes whether the parameter related to lung injury satisfies a second preset rule, which is the same or different from the first preset rule mentioned above. When the second preset rule is the same as the first preset rule, the processor 110 can control the display 120 to display whether each parameter related to lung injury is a parameter that causes the risk of lung injury to the ventilated object, thereby helping the user to adjust the control parameter for ventilation in a targeted manner. Specifically, the processor 110 can control the display 120 to display whether each parameter related to lung injury exceeds the corresponding preset threshold.

In the example in FIG. 2, two different forms of progress bar (shown as different patterns in FIG. 2) indicate whether each parameter related to lung injury exceeds the preset threshold. Specifically, the value range of the driving pressure (Pdrive) is 0-50, and its preset threshold range is 5-15. The current monitoring value for the driving pressure is 20, which exceeds the preset threshold range of driving pressure. Therefore, the progress bar in the first form represents the numerical value of driving pressure. The value range of tidal volume/ideal body weight (TV/IBW) is 0-20, the preset threshold range is 6-12, and the current monitoring value for TV/IBW is 6, which does not exceed the preset threshold range of TV/IBW, so that the second form of progress bar represents the numerical value of TV/IBW. At the same time, the preset threshold range can also be displayed through the progress bar for the user to compare.

Similarly, the value range of end-inspiratory transpulmonary pressure (PtpI) is 0-30, the preset threshold value is 15, and the current monitoring value for PtpI is 10, which does not exceed the preset threshold value of PtpI. Therefore, the second form of progress bar represents the numerical value of PtpI. The value range of end-expiratory transpulmonary pressure (PtpE) is -15-15, and the preset threshold range is -5-5. The current monitoring value for PtpE is 0, which does not exceed the preset threshold of PtpE. Therefore, the second form of progress bar represents the numerical value of PtpE. The value range of mechanical power (MP) is 0-30, the preset threshold value is 15, and the current monitoring value for MP is 16, which exceeds the preset threshold range of mechanical power. Therefore, the progress bar in the first form represents the numerical value of mechanical power.

In further embodiments, when the processor 110 determines that a certain parameter related to lung injury exceeds the corresponding preset threshold, the numerical value or graphic configured to represent the parameter related to lung injury, can be displayed with the same graphic feature as that of the preset graphic, such as the same color, brightness, pattern, etc., so as to indicate the user of the parameter related to lung injury that causes the risk of lung injury. For example, in the example of FIG. 2, because the monitoring values for the driving pressure and mechanical power exceed the corresponding preset threshold ranges, the processor 110 determines that the ventilated object is at the risk of lung injury, controls the display 120 to display information about that the ventilated object is at the risk of lung injury through the dynamic pulmonary graphic, and controls the display 120 to display information about that the driving pressure and mechanical power exceed the preset thresholds through the progress bars. In practical applications, the dynamic pulmonary graphic and the progress bars of driving pressure and mechanical power are displayed in red.

In one embodiment, the processor 110 is further configured to control the display 120 to display a trend chart of at least one parameter related to lung injury, so as to show a trend of change of at least one parameter related to lung injury within a preset time range, which is helpful for the user to understand the change of ventilation state. Wherein, the trend chart of at least one parameter related to lung injury can be the trend chart of the parameter related to lung injury that satisfies the first preset rule. Due to the fact that the parameter related to lung injury that satisfies the first preset rule is the parameter that leads to the risk of lung injury, the user is generally more likely to want to understand its change of trend. Optionally, the trend chart of at least one parameter related to lung injury can also be the trend chart of the parameter related to lung injury, which parameter is specified by the user.

In one embodiment, the processor 110 is further configured to obtain a factor of the ventilated object, which factor is related to pneumodynamics state, determine the pneumodynamics state of the ventilated object according to the factor related to pneumodynamics state, and control the display 120 to present the pneumodynamics state of the ventilated object through the dynamic pulmonary graphic. The pneumodynamics state refers to mechanical states of various parts of the respiratory system of the ventilated object during the mechanical ventilation. The pneumodynamics state can reflect whether the respiration of the ventilated object is laborious.

For example, the factor related to pneumodynamics state includes at least one of: compliance of respiratory system, airway resistance, compliance of lung(s), and compliance of chest wall. Wherein, compliance describes elastic characteristics of various parts of the respiratory system, which can be either static compliance or dynamic compliance. Compliance refers to an expandability of elastic tissue under external force. Wherein, compliance of lung(s) refers to a change in pulmonary volume caused by a change in unit pressure, represents an impact of change in thorax pressure on the pulmonary volume, and reflects the difficulty of change in the lungs under the external force. Compliance of chest wall refers to a change in the pulmonary volume caused by a change in unit pressure which acts on the chest wall, reflects the elastic resistance of the chest wall. Compliance of respiratory system is determined by both compliance of lung(s) and compliance of chest wall.

Airway resistance refers to a pressure difference generated by a unit flow rate in the airway, which can better reflect an obstruction of the airway and assist in determining whether the cause for the decrease of the pulmonary ventilation function comes from the airway. Airway resistance can include at least one of: inspiratory resistance (Ri) and expiratory resistance (Re). Bronchial asthma, Emphysema and obstructive ventilation dysfunction can cause increased airway resistance. In addition, the obstruction of mechanical ventilation pipe may also cause increased airway resistance.

The pneumodynamics state of the ventilated object can be determined by a threshold value of the factor related to pneumodynamics state and presented on the dynamic pulmonary graphic. By way of example, the pneumodynamics state of the ventilated object can be presented by at least one of the airway and the contour in the dynamic pulmonary graphic. In addition, different pneumodynamics state related factors can correspond to different graphic features of the dynamic pulmonary graphic. For example, if the airway resistance exceeds the preset threshold, the airway part of the dynamic pulmonary graphic is displayed with a specific graphic feature. If the compliance exceeds the preset threshold, the contour of the dynamic pulmonary graphic is displayed with a specific graphic feature. Dynamic pulmonary graphic can present the pneumodynamics state of the ventilated object through one or more graphic features of color, brightness and transparency. In practical applications, the type(s) of graphic feature(s) configured to present the pneumodynamics state of the ventilated object can be designed according to actual requirements, and there are no specific limitations here. The pneumodynamics state can also be presented in the form of numerical annotation on the dynamic pulmonary graphic, as shown in FIG. 2.

In one embodiment, the processor 110 can also present a trend of change of the factor related to pneumodynamics state over a certain period of time. As shown in FIG. 2, the display 120 shows the trend of change of airway resistance (R), of compliance (C) and of end-respiration carbon dioxide (EtCO2) on the left side of the dynamic pulmonary graphic.

In one embodiment, the processor 110 is further configured to determine whether an abnormality of airway, such as phlegm accumulation, exists in the ventilated object. After confirming that the ventilated object has the abnormality of airway, the processor 110 is further configured to control the display 120 to display the abnormality of airway through the dynamic pulmonary graphic. The display 120 may display the abnormality of airway through the airway part of the dynamic pulmonary graphic, such as displaying the airway in a specific color, or displaying a specific graphical marker in the airway. Furthermore, when displaying the abnormality of airway through the dynamic pulmonary graphic, a suggestion for treating the abnormality of airway, can also be displayed, which is helpful for the user to treat the abnormality of airway. Different types of abnormality of airway can correspond to different treatment suggestions. For example, if the abnormality of airway is phlegm accumulation, a treatment suggestion of subglottic suction can be displayed. Optionally, the display 120 can also display the abnormality of airway independently, without displaying the treatment suggestion for abnormality of airway, or display treatment suggestion for abnormality of airway independently, without displaying the abnormality of airway through the dynamic pulmonary graphic.

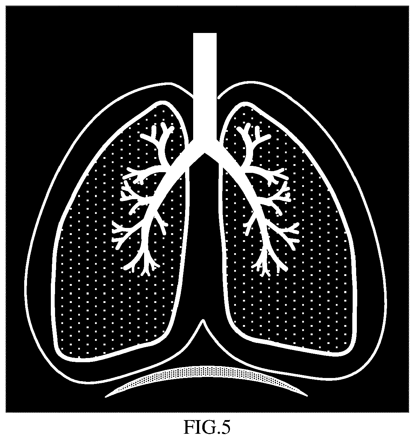

When the parameter related to lung injury includes an esophageal pressure parameter, the processor 110 can also be configured to display a dynamic thoracic graphic around the dynamic pulmonary graphic according to the esophageal pressure parameter, as shown in FIG. 5. The dynamic thoracic graphic is configured to characterize the compliance of chest wall. The dynamic thoracic graphic can be drawn according to the shape of the human thorax, and the dynamic thoracic graphic can be displayed at the periphery of the dynamic pulmonary graphic according to the relative position between the lung and the thorax. The dynamic thoracic graphic can be a two-dimensional graphic or a three-dimensional graphic surrounding the dynamic pulmonary graphic. Exemplarily, the compliance of chest wall can be represented by the graphic feature, such as contour size, contour thickness, color, brightness, etc., of the dynamic thoracic graphic .

Compliance refers to an expandability of elastic tissue under external force. Wherein the compliance of chest wall refers to a change in the pulmonary volume caused by a change in unit pressure which acts on the chest wall, reflects the elastic resistance of the chest wall. The compliance of chest wall can be calculated according to tidal volume and esophageal pressure, so when there are monitoring parameters for esophageal pressure, a compliance parameter of chest wall can be obtained according to the monitoring parameters for esophageal pressure, and the dynamic thoracic graphic can be displayed according to the corresponding relationship between the compliance parameter of chest wall and the dynamic thoracic graphic. Optionally, the measurement of the compliance of chest wall requires complete relaxation of the chest wall muscles, which is difficult to achieve. Therefore, compliance of lung(s) and the total compliance can be measured, and compliance of chest wall can be obtained by calculating the difference between the compliance of lung(s) and the total compliance.

For example, the processor 110 can also configure a preset threshold range for the compliance of chest wall based on the normal compliance of chest wall. If the compliance of chest wall of the ventilated object exceeds the preset threshold range for the compliance of chest wall, it indicates that the compliance of chest wall is too high, and vice versa, it indicates that the compliance of chest wall is too low. If the compliance of chest wall of the ventilated object is within the preset threshold range for the compliance of chest wall, it indicates that the compliance of chest wall is normal. The processor 110 can control the display 120 to display the dynamic thoracic graphic in different forms, according to the three situations that the compliance of chest wall is too high, too low or normal, so that the user can determine the compliance of chest wall of the ventilated object according to the dynamic thoracic graphic.

For example, in the inspiratory phase of mechanical ventilation, the pressure in the thorax gradually increases, the processor 110 can control the display 120 to gradually deepen the color of the dynamic thoracic graphic, and in the expiratory phase of mechanical ventilation, the pressure in the thorax gradually decreases, and the processor 110 can control the display 120 to gradually lighten the color of the dynamic thoracic graphic. At the same time, at different phases, the processor 110 can also control the display 120 to change contour sizes of the dynamic pulmonary graphic and the dynamic thoracic graphic relatively.

In one embodiment, the processor 110 can also determine the state of spontaneous respiration of the ventilated object, and control the display 120 to present the state of spontaneous respiration of the ventilated object through the dynamic pulmonary graphic.

For example, the processor 110 can control the display 120 to present the state of spontaneous respiration of the ventilated object through a diaphragmic graphic. As shown in FIG. 4, the dynamic pulmonary graphic includes the diaphragmic graphic, and presenting the state of spontaneous respiration of the ventilated object through a diaphragmic graphic includes presenting that the ventilated object has spontaneous respiration through the diaphragmic graphic. When the processor 110 determines that the ventilated object has spontaneous respiration, the processor 110 controls the display 120 to display the diaphragmic graphic shown in FIG. 4. When the processor 110 determines that the ventilated object does not have spontaneous respiration, the processor 110 controls the display 120 not to display the diaphragmic graphic, but to display the dynamic pulmonary graphic without the diaphragmic graphic as shown in FIG. 2. According to whether the dynamic pulmonary graphic includes the diaphragmic graphic, the user can intuitively know whether the ventilated object has spontaneous respiration.

Furthermore, the diaphragmic graphic can also be configured to present a strength of the spontaneous respiration of the ventilated object. For example, the strength of the spontaneous respiration of the ventilated object can be presented through any one or more graphic features, such as color, shade, transparency, brightness, and flicker speed of the diaphragmic graphic. This embodiment does not limit the specific graphic feature configured to present the strength of spontaneous respiration.

When the processor 110 determines that the ventilated object has spontaneous respiration, the processor 110 can further determine a percentage of spontaneous respiration of the ventilated object and control the display 120 to display the percentage of spontaneous respiration of the ventilated object. Wherein, the percentage of spontaneous respiration is the percentage of spontaneous respiration rate in a total respiration rate. The display 120 can display the percentage of spontaneous respiration through the diaphragmic graphic, as well as can display the percentage of spontaneous respiration through a numerical or graphical display.

Optionally, the diaphragmic graphic is further configured to present a state of diaphragm injury. The state of diaphragm injury can include whether the diaphragm is injured and the degree of diaphragm injury. The processor 110 can control the display 120 to display the diaphragmic graphic when determining that the spontaneous respiration exists in the ventilated object, and to present the state of diaphragm injury through the diaphragmic graphic. The processor 110 can also control the display 120 to display the diaphragmic graphic regardless of whether the ventilated object has the spontaneous respiration, and to present the state of diaphragm injury through the diaphragmic graphic.

In summary, the medical device system for displaying a state of mechanical ventilation, according to embodiments of this disclosure, visualizes the risk of lung injury for the ventilated object through a preset graphic, thereby guiding the user to reasonably adjust ventilation parameter and maintain ventilation while reducing the lung injury.

On the other hand, an embodiment of this disclosure provides a medical device system for displaying a state of mechanical ventilation . Continuing to refer to FIG. 1, the medical device system 100 includes a processor 110 and a display 120; wherein the display 120 is configured to display a state of mechanical ventilation of a ventilated object during the mechanical ventilation; and the processor 110 is configured to control the display 120 to display, on a display interface, a dynamic pulmonary graphic, wherein the dynamic pulmonary graphic is configured to characterize the state of mechanical ventilation of a ventilated object during the mechanical ventilation, to acquire a state of diaphragm injury of the ventilated object, and to control the display 20 to display, below the dynamic pulmonary graphic, a diaphragmatic graphic, wherein the diaphragmatic graphic is configured to characterize the state of diaphragm injury. The specific embodiment may refer to FIG.4.

Wherein, the diaphragm injury refers to diaphragmatic dysfunction caused by diaphragmatic immobilization and unloaded state during mechanical ventilation. The injury caused by mechanical ventilation to the diaphragm is mainly manifested in a decrease in diaphragm contraction function and atrophy of diaphragm fibers. The state of diaphragm injury of this embodiment can include an actual state of diaphragm injury obtained through measurement, as well as a risk of diaphragm injury obtained through assessment.

For example, the processor 110 can acquire a monitoring parameter for ventilation or a control parameter for ventilation, which is related to the diaphragm injury, and assess the state of diaphragm injury through the monitoring parameter for ventilation or the control parameter for ventilation, which is related to the diaphragm injury. The diaphragm injury related monitoring parameter for ventilation or the diaphragm injury related control parameter for ventilation can include some or all of the above parameters related to lung injury, as well as transdiaphragmatic pressure. Optionally, the processor 110 can also acquire ultrasound image of the diaphragm, assess a graphic feature, such as a diaphragm thickness according to the ultrasound image, and obtain the state of diaphragm injury. For example, the state of diaphragm atrophy can be obtained through the diaphragm thickness. The processor 110 can also directly obtain the assessment result of the state of diaphragm injury. This embodiment does not limit the method of obtaining the state of diaphragm injury of the ventilated object.

The diaphragmic graphic is configured to present the state of diaphragm injury. The state of diaphragm injury can include whether the diaphragm is injured and the degree of diaphragm injury. For example, if the processor 110 determines that the diaphragm is injured, the display displays a diaphragmic graphic. If the processor 110 determines that the diaphragm is not injured, the diaphragmic graphic may not be displayed. The user can quickly determine whether the diaphragm is injured based on whether the diaphragmic graphic is displayed. The processor 110 can also present the state of the diaphragm injury through the diaphragmic graphic with different graphic features. The diaphragmic graphic can also display the degree of diaphragm injury, which can be presented through one or more graphic features such as color, brightness, and transparency. For example, the higher the degree of diaphragm injury, the darker the color. In practical applications, which type or types of graphic features are configured to present the degree of diaphragm injury can be designed according to actual requirements, and there are no specific limitations herein.

The dynamic pulmonary graphic is configured to present the ventilation state of the ventilated object. The ventilation state can include whether the ventilated object is at the risk of lung injury, the type and degree of the risk of lung injury, and the pneumodynamics state. The specific details of the dynamic pulmonary graphic can be referred to above, and are not repeated here.

The medical device system for displaying a state of mechanical ventilation, according to the embodiment of this disclosure visually presents the ventilation state of the ventilated object through the dynamic pulmonary graphic, and visually presents the state of diaphragm injury through the diaphragmic graphics, which helps the user to reasonably set the ventilation parameter.

On the other hand, the embodiment of this disclosure provides a medical device system for displaying a state of mechanical ventilation. Continuing to refer to FIG. 1, the medical device system 100 includes a processor 110 and a display 120, the display 120 is configured to display the state of mechanical ventilation of the ventilated object during the mechanical ventilation, and the processor 110 is configured to control the display 120 to display, on a display interface, a dynamic pulmonary graphic, wherein the dynamic pulmonary graphic is configured to characterize the state of mechanical ventilation of a ventilated object during the mechanical ventilation. The state of mechanical ventilation at least includes an abnormality of airway and/or a treatment suggestion for the abnormality of airway.

Specifically, the processor 110 is further configured to determine whether the ventilated object has an abnormality of airway, such as phlegm accumulation. After determining that the ventilated object has the abnormality of airway, the processor 110 controls the display 120 to display the abnormality of airway through the dynamic pulmonary graphic. The display 120 may display the abnormality of airway through an airway part of the dynamic pulmonary graphic, such as displaying the airway in a specific color, or displaying a specific graphical marker in the airway. Furthermore, when displaying the abnormality of airway through the dynamic pulmonary graphic, a treatment suggestion for the abnormality of airway can also be displayed, which is helpful for the user to process the abnormality of airway. Different types of abnormality of airway can correspond to different treatment suggestions. For example, if the abnormality of the airway is phlegm accumulation, the treatment suggestion of subglottic suction can be displayed. Optionally, the display 120 can display the abnormality of airway independently, without displaying the treatment suggestion for the abnormality of airway, or can display the treatment suggestion for the abnormality of airway independently, without displaying the abnormality of airway through dynamic pulmonary graphic.

In addition, the ventilation state presented by the dynamic pulmonary graphic can also include whether the ventilated object is at the risk of lung injury, the type and degree of the risk of lung injury, and the pneumodynamics state. The specific details of the dynamic pulmonary graphic can be referred to above, and are not repeated here.

The medical device system for displaying a state of mechanical ventilation according to the embodiment of this disclosure visually presents at least one of the abnormality of airway and the treatment suggestion for the abnormality of airway through the dynamic pulmonary graphic, which helps the user to quickly and accurately eliminate the abnormality of airway.

Although the exemplary embodiments are described here with reference to the accompanying drawings, it should be understood that the aforementioned exemplary embodiments are only illustrative and are not intended to limit the scope of this disclosure. Ordinary technical personnel in this field can make various changes and modifications without deviating from the scope and spirit of this disclosure. All these changes and modifications are intended to be included within the scope of this disclosure as claimed in the attached claims.

Ordinary technical personnel in this field can realize that the units and algorithm steps described in combination with this disclosure can be implemented in electronic hardware, or a combination of computer software and electronic hardware. Whether these functions are executed in hardware or software depends on the specific application and design constraints of the technical solution. Professional technicians may use different methods to implement the described functions for each specific application, but such implementation should not be considered beyond the scope of this disclosure.

In the several embodiments provided in this disclosure, it should be understood that the disclosed devices and methods can be implemented in other ways. For example, the device embodiments described above are only schematic. For example, the division of the units is only a logical functional division, and there may be other division methods in actual implementations. For example, multiple units or components can be combined or integrated into another device, or some features can be ignored or not executed.

The disclosure provided here provides a large number of specific details. However, it can be understood that the embodiments of this disclosure can be practiced without these specific details. In some examples, well-known methods, structures, and techniques are not shown in detail to avoid blurring the understanding of this disclosure.

Similarly, it should be understood that in order to streamline this disclosure and assist in understanding one or more of the various aspects of this disclosure, in the description of exemplary embodiments of this disclosure, the various features of this disclosure are sometimes grouped together into a single embodiment, diagram, or description thereof. However, the method of this disclosure should not be interpreted as reflecting the intention that the claimed protection of this disclosure requires more features than those explicitly recorded in each claim. More precisely, as reflected in the corresponding claims, the inventive point is that the corresponding technical problem can be solved with less than all features of a single disclosed embodiment. Therefore, the claims following the specific implementation method are explicitly incorporated into the specific implementation method, where each claim itself serves as a separate embodiment of this disclosure.

Technicians in this field can understand that, in addition to mutual exclusion between features, any combination can be configured to combine all features disclosed in this disclosure (including accompanying claims, abstract, and drawings), as well as all processes or units of any method or device so disclosed. Unless otherwise explicitly stated, each feature disclosed in this disclosure (including accompanying claims, abstract, and accompanying drawings) may be replaced by alternative features that provide the same, equivalent, or similar purpose.

In addition, those skilled in the art can understand that although some embodiments described herein include certain features rather than other features included in other embodiments, the combination of features of different embodiments means that they are within the scope of this disclosure and form different embodiments. For example, in the claims, any one of the claimed embodiments can be used in any combination.

The various component embodiments of this disclosure can be implemented in hardware, software modules running on one or more processors, or a combination thereof. Technicians in this field should understand that a microprocessor or digital signal processor (DSP) can be used in practice to implement some or all of the functions of some modules according to the embodiments of this disclosure. This disclosure can also be implemented as a partial or complete device program (such as a computer program and a computer program product) for executing the method described herein. The program implementing this disclosure can be stored at a computer-readable medium or can have the form of one or more signals. Such signals can be downloaded from internet websites, provided on carrier signals, or in any other form.

It should be noted that the above embodiments illustrate this disclosure rather than limit it, and those skilled in the art can design alternative embodiments without departing from the scope of the attached claims. In the claims, any reference symbol between parentheses should not be constructed as a restriction on the claims. This disclosure can be implemented with the help of hardware consisting of several different components and with the help of appropriately programmed computers. Among the unit claims that list several devices, several of these devices can be specifically embodied through the same hardware item. The use of words first, second, and third does not indicate any order. These words can be interpreted as names.

The above is only the specific implementation method or explanation of the specific implementation method of this disclosure. The protection scope of this disclosure is not limited to this. Any technical personnel familiar with the technical field can easily think of changes or replacements within the technical scope disclosed in this disclosure, and those changes or replacements should fall into the protection scope of this disclosure. The protection scope of this disclosure shall be based on the protection scope of the claims.

## Claims

1. A medical device system for displaying a state of mechanical ventilation, comprising a display and a processor; wherein the display is configured for displaying the state of mechanical ventilation;
**characterized in that**, the processor is configured for:
acquiring at least one parameter of a ventilated object, which parameter is related to lung injury of the ventilated object;
determining a risk of lung injury for the ventilated object according to the at least one parameter related to lung injury; and
controlling the display to present, through a preset graphic, the risk of lung injury, when the ventilated object is determined to be at the risk of lung injury.

2. The medical device system according to claim 1, **characterized in that**, the preset graphic comprises a dynamic pulmonary graphic, wherein the display is further configured for presenting, through the dynamic pulmonary graphic, the risk of lung injury.

3. The medical device system according to claim 1 or claim 2, **characterized in that**, the at least one parameter related to lung injury comprises at least one of: a monitoring parameter for ventilation and a control parameter for ventilation.

4. The medical device system according to claim 3, **characterized in that**,
the monitoring parameter for ventilation comprises at least one of: an airway plateau pressure, a driving pressure, a monitoring value for PEEP, a monitoring value for tidal volume, a functional residual volume, a transpulmonary pressure, mechanical power, respiratory power, and an esophageal pressure;
the control parameter for ventilation comprises at least one of: a setting value for tidal volume, an ideal weight, a height, an inspiratory pressure, and a setting value for PEEP.

5. The medical device system according to claim 1 or claim 2, **characterized in that**, determining a risk of lung injury for the ventilated object according to the at least one parameter related to lung injury, comprises:
determining that the ventilated object is at the risk of lung injury, when the at least one parameter related to lung injury satisfies a first preset rule.

6. The medical device system according to claim 5, **characterized in that**, determining that the ventilated object is at the risk of lung injury, when the at least one parameter related to lung injury satisfies a first preset rule, comprises:
determining that the ventilated object is at the risk of lung injury, when the at least one parameter related to lung injury exceeds a corresponding preset threshold.

7. The medical device system according to claim 1 or claim 2, **characterized in that**, presenting, through a preset graphic, the risk of lung injury, comprises:
presenting the risk of lung injury, through at least one graphic feature of color, brightness and transparency in the preset graphic.

8. The medical device system according to claim 2, **characterized in that**, presenting, through the dynamic pulmonary graphic, the risk of lung injury, comprises:
presenting the risk of lung injury, through a graphic feature within the dynamic pulmonary graphic, or
presenting the risk of lung injury, through a graphic feature within a background area of the dynamic pulmonary graphic.

9. The medical device system according to claim 1 or claim 2, **characterized in that**, presenting, through a preset graphic, the risk of lung injury, comprises:
presenting a first graphic feature of the preset graphic, when the ventilated object is determined to be at the risk of lung injury; and
presenting a second graphic feature of the preset graphic, when the ventilated object is determined not to be at the risk of lung injury.

10. The medical device system according to claim 1 or claim 2, **characterized in that**, presenting, through a preset graphic, the risk of lung injury, comprises:
presenting a graphic feature of the preset graphic, which feature corresponds to the at least one parameter related to lung injury, according to a corresponding relationship between the graphic feature of the preset graphic and the at least one parameter related to lung injury.

11. The medical device system according to claim 10, **characterized in that**, the corresponding relationship between the graphic feature of the preset graphic and the at least one parameter related to lung injury, comprises:
a corresponding relationship between a graphic feature of a whole area of the preset graphic and the at least one parameter related to lung injury; and/or
a corresponding relationship between a graphic feature of a partial area of the preset graphic and the at least one parameter related to lung injury.

12. The medical device system according to claim 10, **characterized in that**, each parameter related to lung injury corresponds to one graphic feature; or
each type of parameter(s) related to lung injury corresponds to one graphic feature.

13. The medical device system according to claim 1 or claim 2, **characterized in that**, the processor is further configured for:
determining a degree of the risk of lung injury, according to the at least one parameter related to lung injury; and
presenting, through the preset graphic, the degree of the risk of lung injury.

14. The medical device system according to claim 13, **characterized in that**, presenting, through the preset graphic, the degree of the risk of lung injury, comprises:
displaying, in different colors, different shades of color and/or different transparencies, the preset graphic, which corresponds to different degrees of the risk of lung injury.

15. The medical device system according to claim 1 or claim 2, **characterized in that**, the processor is further configured for:
controlling the display to display, in an adjacent area of the preset graphic, the at least one parameter related to lung injury, through a numerical value or a chart.

16. The medical device system according to claim 15, **characterized in that**, the processor is further configured for:
presenting, through the numerical value or the chart, a state of the at least one parameter related to lung injury, wherein the state of the at least one parameter related to lung injury comprises whether the at least one parameter related to lung injury satisfies a second preset rule.

17. The medical device system according to claim 1 or claim 2, **characterized in that**, the processor is further configured for:
controlling the display to display a trend chart for the at least one parameter related to lung injury.

18. The medical device system according to claim 2, **characterized in that**, the processor is further configured for:
acquiring a factor of the ventilated object, which factor is related to a pneumodynamics state of the ventilated object;
determining the pneumodynamics state of the ventilated object, according to the factor related to pneumodynamics state; and
controlling the display to present, through the dynamic pulmonary graphic, the pneumodynamics state.

19. The medical device system according to claim 18, **characterized in that**, the factor related to pneumodynamics state comprises at least one of: compliance of respiratory system, airway resistance, compliance of lung(s), and compliance of chest wall.

20. The medical device system according to claim 18, **characterized in that**, presenting, through the dynamic pulmonary graphic, the pneumodynamics state, comprises:
presenting, through an airway and/or a contour, which are/is within the dynamic pulmonary graphic, the pneumodynamics state.

21. The medical device system according to any one of claims 18-20, **characterized in that**, the processor is further configured for:
displaying, through the dynamic pulmonary graphic, an abnormality of airway and/or a treatment suggestion for an abnormality of airway, when the ventilated object is determined to have the abnormality of airway.

22. The medical device system according to claim 2, **characterized in that**, when the at least one parameter related to lung injury comprises an esophageal pressure parameter, the processor is further configured for:
displaying, at a periphery of the dynamic pulmonary graphic, a dynamic thoracic graphic, according to the esophageal pressure parameter;
wherein the dynamic thoracic graphic is configured for characterizing compliance of chest wall.

23. The medical device system according to claim 2, **characterized in that**, the processor is further configured for:
determining a state of spontaneous respiration of the ventilated object; and
controlling the display to present, through the dynamic pulmonary graphic, the state of spontaneous respiration.

24. The medical device system according to claim 23, **characterized in that**, the dynamic pulmonary graphic comprises a diaphragmatic graphic;
wherein presenting, through the dynamic pulmonary graphic, the state of spontaneous respiration, comprises:
presenting, through the diaphragmatic graphic, that the ventilated object has the spontaneous respiration.

25. The medical device system according to claim 24, **characterized in that**, the diaphragmatic graphic is further configured for presenting a strength of the spontaneous respiration of the ventilated object.

26. The medical device system according to claim 24, **characterized in that**, presenting, through the dynamic pulmonary graphic, the state of spontaneous respiration, further comprises:
controlling the display to display a percentage of the spontaneous respiration of the ventilated object.

27. The medical device system according to any one of claims 23-26, **characterized in that**, the diaphragmatic graphic is further configured for presenting a state of diaphragm injury.

28. The medical device system according to claim 1 or claim 2, **characterized in that**, presenting, through a preset graphic, the risk of lung injury, comprises:
controlling the display to display proportions of various types of lung injury in the risk of lung injury.

29. The medical device system according to claim 1 or claim 2, **characterized in that**, presenting, through a preset graphic, the risk of lung injury, comprises:
displaying in real-time, in a preset display area of the display, the preset graphic; or
displaying, on a pop-up window of the display, the preset graphic, when an instruction for displaying the preset graphic is received.

30. A medical device system for displaying a state of mechanical ventilation, comprising a display and a processor; wherein the display is configured for displaying the state of mechanical ventilation;
**characterized in that**, the processor is configured for:
controlling the display to display, on a display interface, a dynamic pulmonary graphic, wherein the dynamic pulmonary graphic is configured for characterizing a state of mechanical ventilation of a ventilated object during mechanical ventilation;
acquiring a state of diaphragm injury of the ventilated object; and
controlling the display to display, below the dynamic pulmonary graphic, a diaphragmatic graphic, wherein the diaphragmatic graphic is configured for characterizing the state of diaphragm injury.

31. A medical device system for displaying a state of mechanical ventilation, comprising a display and a processor; wherein the display is configured for displaying the state of mechanical ventilation;
**characterized in that**, the processor is configured for:
controlling the display to display, on a display interface, a dynamic pulmonary graphic, wherein the dynamic pulmonary graphic is configured for characterizing a state of mechanical ventilation of a ventilated object during mechanical ventilation; wherein the state of mechanical ventilation of the ventilated object at least comprises an abnormality of airway and/or a treatment suggestion for an abnormality of airway.
